# EUROPEAN PATENT SPECIFICATION

(11) **EP 2 486 946 B1**
(45) Date of publication and mention of the grant of the patent: **03.06.2015**
(21) Application number: 10822132.6
(22) Date of filing: 08.10.2010
(51) Int. Cl.: A61L 17/00

(54) **BIORESORBABLE SUTURE THREAD**
BIOLOGISCH ABBAUBARER NÄHFADEN
FIL DE SUTURE BIORÉSORBABLE

(30) Priority: 09.10.2009 JP 2009235320
(43) Date of publication of application: 15.08.2012
(73) Proprietor: Totai Co., Ltd., Taito-ku Tokyo 110-0005 (JP)
(72) Inventor: YOSHIKAWA, Masatoshi, Kakogawa-shi Hyogo 675-0023 (JP); KAJITA, Hideki, Kakogawa-shi Hyogo 675-0023 (JP); NOGUCHI, Takeshi, Taito-ku Tokyo 110-0005 (JP); KASAI, Juichi, Taito-ku Tokyo 110-0005 (JP)
(74) Representative: Schrell, Andreas
(86) International application number: PCT/JP2010/067749
(87) International publication number: WO 2011/043462

(56) References cited:
- EP-A1- 0 051 421
- WO-A1-2011/042971
- WO-A2-2005/002510
- JP-A- 5 051 401
- JP-A- 5 056 792
- JP-A- 5 064 595
- JP-A- 58 183 169
- JP-A- 62 078 215
- JP-A- 2009 247 824
- DATABASE WPI Week 199506 Thomson Scientific, London, GB; AN 1995-041422 XP002722541, & JP H06 322181 A (KURARAY CO LTD) 22 November 1994 (1994-11-22) -& DATABASE EPODOC [Online] EUROPEAN PATENT OFFICE, THE HAGUE, NL; XP002722542, -& JP H06 322181 A (KURARAY CO) 22 November 1994 (1994-11-22)
- DATABASE WPI Week 200974 Thomson Scientific, London, GB; AN 2009-Q70027 XP002722543, & JP 2009 247824 A (THERMOSTABLE ENZYME LAB CO LTD) 29 October 2009 (2009-10-29) -& DATABASE EPODOC [Online] EUROPEAN PATENT OFFICE, THE HAGUE, NL; XP002722544, & JP 2009 247824 A (THERMOSTABLE ENZYME LAB CO LTD; TOTAI CO LTD) 29 October 2009 (2009-10-29)
- DATABASE WPI Week 200901 Thomson Scientific, London, GB; AN 2009-A16465 XP002722545, & JP 2008 264147 A (FUJI FILM CO LTD) 6 November 2008 (2008-11-06)

## Description

### Technical Field

The present invention relates to a suture which is degraded and absorbed in a living body.

### Background Art

Surgical sutures are classified into absorbable sutures which are degraded and absorbed in a living body and unabsorbable sutures which are not degraded or absorbed in a living body. These absorbable suture and unabsorbable suture are used depending on a surgical site, a period of being sutured, and the like. Though the unabsorbable suture has a stable tensile strength required for surgery, this has the drawback that it is necessary to reoperate so as to remove the suture left in the body as a foreign matter after surgery.

On the other hand, when using the absorbable suture, it is unnecessary to reoperate because it is absorbed in a living body after surgery. However, external force acting on the surgical site differs depending on the surgical site, and therefore, it is necessary to control the temporal variation in the tensile strength (hereinafter referred to as a rate of degradation) of the suture depending on an objective. Specifically, the suture is preferably one which has a predetermined tensile strength to tie the surgical site until the surgical site has cured and of which the rate of degradation is controlled such that the suture is degraded along with the cure of the surgical site. Conventionally, the control of the rate of degradation of the suture has been conducted by controlling the degree of polymerization of a synthetic polymer which is a constituent material of the suture, combining different materials, and using the suture in echinulate form.

For example, Patent reference 1 discloses an absorbable suture constituted of a synthetic polymer having a rate of degradation controlled by adjusting the type of a monomer constituting the synthetic polymer, the ratio of the monomer to be polymerized, and the degree of polymerization (molecular weight).

Further, Patent reference 2 discloses a biodegradable suture in echinulate form. This suture is characterized in that it is constituted using a synthetic polymer synthesized by adjusting the type of monomer and the ratio of the monomer to be polymerized and that the rate of degradation of the suture is controlled by regulating the crystallinity and surface area of the synthetic polymer.

However, it is hardly considered that these methods can easily control the rate of degradation of a suture. For example, it is not easy to regulate the degree of polymerization of a constituent material so as to control the rate of degradation of the suture from the viewpoint of equipment and the like. Further, when the degree of polymerization of a constituent material is reduced in order to increase the rate of degradation of a suture, there may be a problem that the strength required for the suture at the time of suturation is not satisfied. It is therefore desired to develop a method of easily controlling the rate of degradation of a suture without reducing the strength of the suture.

Patent reference 3 discloses a biodegradable suture constituted of a polypeptide or polysaccharide derived from a living body such as a biopolymer. However, no disclosure is found as to the regulation of rate of degradations of these sutures.

An attention is focused on chitin which is aminosaccharides as a bioabsorbable fiber material. For example, Cited reference 4 discloses that a chitin fiber is superior in biocompatibility and has excellent effect as a wound coating agent. It is known that chitin not only is a bioabsorbable material but also has additional effects such as wound cure-promoting effect and hemostatic effect. However, as for the chitin fiber, there is no finding concerning the degradation control, and therefore, the chitin fiber has not been practically used as a suture in spite of its superior characteristics. The utilization of the chitin fiber as a suture is greatly expected from the viewpoint of not only bioabsorbability but also the cure effect which the suture itself has.

### Prior Art Reference

### Patent Reference

Patent reference 1: Jpn. Pat. Appln. KOKAI Publication No. 2001-54563
Patent reference 2: Jpn. Pat. Appln. KOKAI Publication No. 2008-114074
Patent reference 3: Jpn. Pat. Appln. KOKAI Publication No. 2008-264147
Patent reference 4: Japanese Patent No. 3046099

JPH06322181 discloses molded articles, including fibres, made from biodegradable polymers including chitosan, starch or cellulose and comprising polymer degrading enzymes or microorganisms.

### Summary of Invention

### Technical Problem

An object of the present invention is to provide a bioabsorbable suture which includes chitin, chitosan, or derivatives thereof as a raw material and has a rate of degradation that may easily be controlled depending on an objective and an application.

### Solution to Problem

The present invention relates to bioabsorbable sutures according to the independent claims 1-3.

According to the one aspect, there is provided a bioabsorbable suture having a controlled rate of degradation, where the suture is manufactured from a monofilaments or multifilaments spun from a spinning raw solution containing one or two or more component(s) selected from the group consisting of chitin, chitosan, and derivatives thereof and a degrading enzyme for the component(s), and the degrading enzyme exists on a surface of the suture and in the suture.

### Advantageous Effects of Invention

According to the present invention, a bioabsorbable suture can be provided which includes chitin, chitosan, or derivatives thereof as a raw material and has a rate of degradation that may easily be controlled depending on an objective and an application.

### Description of Embodiments

An embodiment will be explained in detail.

A bioabsorbable suture according to the present invention is characterized in the suture is loaded with an enzyme which degrades the constituent material of a fiber in the process of manufacturing the suture. In such a constituent, a suture which is biodegradable and maintains the strength required for the suture at the time of surgery can be obtained. Further, the rate of biodegradation can be controlled by regulating the type, quantity, concentration, activity, and the like of the enzyme used to load the fiber. According to the present invention, the rate of degradation of the suture can be controlled more easily than that in conventional methods.

The suture of the present invention is characterized by containing chitin, chitosan, or the derivatives thereof (hereinafter referred to also as chitins) as a raw material. Chitin, chitosan or the derivatives thereof may be contained either singly or with a combination of two or more. The above chitin is aminopolysaccharides existing as an organic framework material of Arthropods, Annelids, and Molluscas and is also referred to as poly-N-acetylglucosamine. Generally, the exoskeletons and the like of these Crustacea are subjected to acid treatment using hydrochloric acid or the like and to alkali treatment using caustic soda or the like to decalcify and deproteinize, thereby obtaining chitin. Chitin used in the present invention is one produced under process conditions usually adopted and has a solid form such as a powder. Chitosan is obtained by alkali-hydrolyzing a side chain of chitin.

As a chitin derivative, acylated chitin, carboxylated chitin, phosphorylated chitin, and the like may be used. Further, as the derivative of chitosan, acylated chitosan, carboxylated chitosan, and the like may be used.

Chitins have the characteristics that the biocompatibility is high and the inflammatory response rarely occurs even when chitins are taken into a living body. Chitins also have a wound cure-promoting effect, hemostatic effect, and the like. Therefore, such additional effects on a living body are expected by using a chitin fiber as a suture.

A degrading enzyme used to load a chitin fiber is not particularly limited as long as it can be activated at the body temperature inside the living body to degrade chitins and is a pharmaceutically acceptable enzyme. However, enzymes are usually denatured at a temperature ranging from 50 to 60°C or more to lose the activity. There is the case where processing temperature exceeds this temperature range in the manufacture stage of a suture. This is because a suture raw solution to be used in manufacture of filaments constituting the suture has a high viscosity and it is therefore desirable to carry out the solidification, the drawing and the drying at a temperature range from 40 to 90°C from the viewpoint of productivity and qualitative stability. Therefore, the degrading enzyme used in the present invention is preferably a heat-resistant enzyme which is not deactivated and is stable even at 90°C.

The heat-resistant enzyme means an enzyme which is not deactivated in a high-temperature condition (70 to 100°C) and maintains an activity, and is superior in long-term storage stability even at a temperature close to normal temperature. Therefore, when such a heat-resistant enzyme is used, the function of the bioabsorbable suture of the present invention can be maintained even in the case of performing high-temperature treatment during the course of manufacturing a suture. Further, a suture which has excellent storage stability, that is, can be stored at normal temperature as a product, can be obtained. Therefore, there is no need to store the product under a cold condition, and the suture is significantly improved in usability.

Examples of the enzyme which can degrade chitin and the derivative thereof include chitinase. From the reason described above, the above chitinase is preferably a heat-resistant chitinase. For example, a heat-resistant chitinase and the like derived from Pyrococcus furiosus may be used. In the case of a suture using chitosan and the derivative thereof as its constituent material, chitosanase may be used as the above enzyme.

The following explanations are furnished as to a method of loading the suture with the degrading enzyme.

In the first embodiment, loading the fiber with the degrading enzyme can be performed by spinning filaments from a spinning raw solution containing chitins and a degrading enzyme for these chitins. A suture is obtained in which the degrading enzyme is intermingled on a surface and inside thereof by manufacturing the suture from the filaments obtained in this manner. The above filament may be a monofilament or multifilament.

The spinning may be carried out by general wet spinning. Wet spinning is a method to obtain a fiber by ejecting a spinning raw solution into a coagulating solution. Chitin, chitosan or the derivatives thereof are dissolved in a known mixture solvent containing each component in an appropriate ratio, followed by filtration and degassing treatment to obtain a transparent high-viscosity solution, and the obtained solution is used as a spinning raw solution. The chitins are preferably dissolved in an amount of 5 to 10% by weight based on the solvent. As the above mixture solvent, for example, a mixture solution of dimethylacetamide and lithium chloride, a mixture solution of N-methylpyrrolidone and lithium chloride, a mixture solvent of trichloroacetic acid and hydrocarbon halide, or the like may be used, though the mixture solvent is not limited as long as it is generally used. A degrading enzyme for chitin, chitosan, or the derivatives thereof is mixed in advance in the spinning raw solution obtained in this manner. The degrading enzyme is preferably added such that the active unit is 0.0001 to 10 U/g based on the spinning raw solution.

Here, the active unit of an enzyme will be explained. 1 U is defined as an enzymatic activity level at which 1 µmol of N-acetylglucosamine or glucosamine is freed for one minute. A method of measuring the enzymatic activity will be explained below.

Substrate solution: a chitin powder or chitosan powder is added to be an amount of 0.5% by weight in 200 nM of acetic acid buffer solution (pH 5.6) to suspend.

Enzyme solution: an enzyme which is subject to measurement of activation is dissolved to be an amount of 0.8 mg/ml in distilled water.

Measurement protocol:
(1) 1 ml of the substrate solution is preincubated at 37°C for 5 to 10 minutes in a test tube.
(2) Then, 0.2 ml of an enzyme solution is added in the test tube, which is then incubated at 37°C for 1 hour under a mild shaking condition.
(3) After the incubated solution is ice-cooled, 1000 µl of a DMAB (paradimethylaminobenzaldehyde) reagent is added in the solution. The mixture is heated at 37°C for 20 minutes, and then, absorbance at 585 nm is measured to measure the increase in reduction terminal, thereby quantitatively measuring the amount of free N-acetylglucosamine or glucosamine. In this case, the DMAB reagent is produced by adding 10 g of DMAB in 100 ml of acetic acid containing 12.5% by weight of 10 N hydrochloric acid and is diluted 10 times with acetic acid just before the use. The enzymatic activity is calculated from the amount of freed N-acetylglucosamine or glucosamine obtained.

A method of mixing the degrading enzyme may be performed using a known mixer such as a homomixer and in this case, any of batch mixing or continuous mixing may be adopted.

In succession, a quantitative transport unit such as a gear pump is used to transport a fixed amount of the above spinning raw solution to a spinneret and the spinning raw solution is extruded from the spinneret at a fixed rate into a coagulating solution to draw, thereby producing filaments. The obtained filaments are wound around a reel, and are, in the case of a multifilament, further woven. The above coagulating solution may be a solvent in which chitins are insoluble and water is generally used. The mixing of the degrading enzyme may be carried out while the spinning raw solution is transported to the spinneret.

The amount, concentration, and activity of the degrading enzyme loaded to the suture can be controlled by controlling the amount, concentration, and activity of the degrading enzyme to be mixed in the spinning raw solution. According to the above loading method, the rate of degradation of the suture can be easily controlled. Generally, the rate of degradation of the suture is increased with the increase in the amount, concentration, and activity of the degrading enzyme loaded to the suture.

In a second embodiment, loading the fiber with the degrading enzyme is performed by applying a coating agent containing the degrading enzyme onto the surface of filaments spun from the spinning raw solution containing chitins. A suture of which at least the surface is applied with the degrading enzyme is obtained by manufacturing the suture from the filaments applied with the coating agent.

A method of spinning the filaments is the same as that explained in the above first embodiment. Filaments may be spun from a spinning raw solution in which the degrading enzyme is mixed in advance as in the above first embodiment and a coating agent containing the degrading enzyme may be further applied onto the surfaces of the obtained filaments. Alternatively, the degrading enzyme may be only applied onto the surfaces of the filaments without mixing it into the spinning raw solution.

The application of the coating agent onto the surfaces of the filaments may be carried out during the course of winding the obtained filaments up or during the course of winding the once wound filaments back. As for a coating method, the coating agent may be applied either by dipping the filaments in the coating agent or by using a known coating machine such as a roll coater. After the coating agent is applied to the filaments, a solvent contained in the above coating agent may be removed by drying if necessary.

As the coating agent, a solution type coating agent prepared by adding a solvent to a base agent formulated with the degrading enzyme to adjust the viscosity thereof or an emulsion-type or gel-type coating agent composed of the degrading enzyme, a base agent, and various solvents may be used. Any material may be used as the base agent which is a constituent of the coating agent as long as it is not harmful for a living body, such as irritation to the skin. For example, hydrophobic base agents such as Vaseline, liquid paraffin, silicone, and animal or vegetable oil and fats, and hydrophilic base agents such as lanoline, stearyl alcohol, cetanol, propylene glycol, and polypropylene glycol may be used either singly or with a combination of two or more. In this case, a surfactant, antibacterial agent, thickener, solvent, and the like may be added if necessary.

The degrading enzyme is preferably added such that the active unit is 0.001 to 100 U/g based on the coating agent. The amount, concentration, and activity of the degrading enzyme loaded to the suture can be controlled by controlling the amount, concentration, and activity of the degrading enzyme to be mixed in the coating agent. According to the above loading method, the rate of degradation of the suture can be easily controlled. Generally, the rate of degradation of the suture is increased with the increase in the amount, concentration, and activity of the degrading enzyme loaded to the suture. Further, when a hydrophobic base material is used as the base agent of the coating agent, this produces the effect of retarding the rate of degradation of the suture. When a hydrophilic base agent is used, on the other hand, the effect of accelerating the rate of degradation is increased with the increase of the degree in hydrophilicity.

In a third embodiment, loading the fiber with the degrading enzyme is performed by attaching the degrading enzyme to the surface of filaments spun from the spinning raw solution containing chitins. A suture with the degrading enzyme attached at least to a surface thereof is obtained by manufacturing the suture from the filaments to which the degrading enzyme is attached.

A method of spinning the filaments is the same as that explained in the above first embodiment. Filaments may be spun from a spinning raw solution in which the degrading enzyme is mixed in advance as in the above first embodiment and the degrading enzyme may be further attached to the surfaces of the obtained filaments. Alternatively, the degrading enzyme may be only attached to the surfaces of the filaments without mixing it in the spinning raw solution.

The attachment of the degrading enzyme onto the surface of the filaments may be carried out during the course of winding the obtained filaments up or during the course of winding the once wound filaments back. The attachment of the degrading enzyme may be carried out by impregnating the filaments with a solution of the enzyme, dispersion solution in which a solid with the enzyme attached thereto is dispersed, or w/o emulsion in which an aqueous solution of the enzyme is dispersed and then by drying the solution to remove a solvent. The impregnation method may be a continuous system or batch system.

The degrading enzyme is preferably added such that the active unit is 0.001 to 100 U/g based on the solution and the like in which the filaments are impregnated. In this case, as in the second embodiment, the rate of degradation of the suture can be easily controlled by regulating the amount, concentration, and activity of the degrading enzyme to be added.

With regard to an absorbable suture superior in biocompatibility, the yarn strength required until the surgical site has cured is different from that required after the surgical site has cured depending on each of the sutured site and the objective. Therefore, it is necessary to control the strength and rate of degradation of the suture depending on the sutured site or the like. Further, in order to improve the workability during surgery, it is also necessary to optimally control the stiffness and tying ability and the like of the suture.

For this, the bioabsorbable suture of the present invention is a multifilament (hereinafter also referred to as a commingled yarn) obtained by combining a fiber made from chitin, chitosan, or the derivatives thereof with a bioabsorbable synthetic polymer fiber and/or a bioabsorbable fiber derived from a naturally-ocurring polymer (excluding fibers made from chitin, chitosan, or the derivatives thereof). By using the commingled yarn a suture having optimum characteristics (for example, strength and rate of degradation) depending on the sutured site can be obtained though the characteristics of the suture which are required are different depending on the sutured site. In the commingled yarn, fibers using chitin, chitosan, or the derivatives thereof as the raw material may be contained either singly or with a combination of two or more.

Any fiber may be used as the above bioabsorbable synthetic polymer fiber as long as it has bioabsorbability. Examples of the polymer fiber include a polylactic acid, polyglycolic acid, poly-p-dioxanone, L-lactide/glycolic acid copolymer, L-lactide/ε-caprolactone copolymer, glycollide/ε-caprolactone copolymer, and glycollide/trimethylene carbonate copolymer. Examples of the bioabsorbable fiber derived from natural polymers include a fiber such as a gelatin fiber, glycosaminoglycan fiber, and carrageenan fiber.

The commingled yarn may be produced by mixing two or more types of spinning raw solutions, each type has different components each other, at the spinneret to spin both solutions simultaneously or by spinning both solutions into different filaments, which are then twisted.

For the commingled yarn the method of loading the fiber with the degrading enzyme is the same as above. For the commingled yarn, an enzyme which degrades other fiber materials constituting the suture may be also loaded to the fiber or only the enzyme which degrades chitins may be loaded to the fiber. When only the enzyme which degrades chitins may be loaded to the fiber, chitin fibers can be degraded earlier than other fibers, and therefore, the rate of degradation of the suture can be controlled also by the ratio occupied by chitin fibers in the suture.

According to the present invention, the rate of degradation of the suture can be controlled by varying, for example, the amount of the enzyme loaded to the suture. According to the present invention, a bioabsorbable suture which is degraded at a desired rate depending on, for example, the sutured site and the objective. Though conventionally, a measures is taken to decrease the degree of polymerization of the constituent material of the suture in order to increase rate of degradation, it is unnecessary to decrease the degree of polymerization in the present invention. If the degree of polymerization is decreased, this causes the problem that the yarn strength is lowered. However, it is unnecessary to decrease the degree of polymerization of the constituent material of the suture in the present invention and the rate of degradation can be controlled in spite of the degree of polymerization. Therefore, a suture having the strength required at the time of surgery is obtained. Further, according to the present invention, the rate of degradation can be controlled by a simple method.

### Examples

The present invention will be explained in more detail by way of Examples, which are, however, not intended to be limiting of the technological scope of the present invention.

### <Comparative Example 1>

A solution in which 8% by weight of a chitin powder (manufactured by Koyo Chemical Co., Ltd.) was dissolved in a dimethylacetamide solution containing 8% by weight of lithium chloride, was prepared. This solution was filtered and defoamed under reduced pressure to make a spinning raw solution. The spinning raw solution was transported at a constant rate by a gear pump and extruded from the spinneret (0.07 mm in diameter, 50 holes) into a coagulating bath (60°C warm water). The extruded chitin fiber was wound at a rate of 5 m per minute. The wound chitin fiber was treated with hot water and washed. The obtained chitin fiber had a single yarn fineness of 2.0 dtex. 50 filaments of the obtained chitin fiber are joined into one bundle and three bundles were twisted to form one suture.

One suture (length: 200 mm) was dipped in a dipping solution at 37°C for 5 minutes, washed, and dried to measure a variation in the tensile strength of the suture with time. The tensile strength was measured using a Tensilon RTF-1325. The composition of the dipping solution was prepared by adding 1 ml of 1 M tris-hydrochloric acid buffer (pH 7.5) to 9 ml of distilled water.

The results of measurement of the tensile strength are shown in Table 1 below.

**Table 1**

| Days elapsed (days) | Tensile strength (N) |
|---|---|
| 0 | 5.9 |
| 3 | 4.8 |
| 7 | 4.4 |

As a result of the above test, it was found that the rate of degradation of the suture of Comparative Example 1 was mild. Though the tensile strength was gradually reduced with time, it could be judged that it was difficult to control the rate of retardation.

### <Example 1>

### (Comparative example) (Suture A)

0.25 µL of heat-resistant chitinase (manufactured by Thermostable Enzyme Laboratory Co., Ltd.) having an active unit of 101 U/ml was added to and mixed with 14 g of a solution prepared by dissolving 8% by weight of the same chitin powder as that used in Comparative Example 1 in the same solution as that used in Comparative Example 1. This enzyme-containing chitin solution was filtered and defoamed under reduced pressure to prepare a spinning raw solution. This spinning raw solution was used to spin in the same manner as in Comparative Example 1, thereby obtaining a chitin fiber having a single yarn fineness of 2.0 dtex. 50 filaments of the obtained chitin fiber are joined into one bundle and three bundles were twisted to form one suture.

### (Suture B)

A suture B was manufactured in the same manner as in the case of the suture A except that the amount of heat-resistant chitinase (manufactured by Thermostable Enzyme Laboratory Co., Ltd.) to be added was 25 µL.

The sutures A and B (each length: 200 mm) obtained above were dipped in a dipping solution having the same composition as that of Comparative Example 1 at 37°C for 5 minutes, washed, and dried, to measure a variation in the tensile strength of the sutures with time. The tensile strength was measured in the same manner as in Comparative Example 1.

The results of measurement of the tensile strength of the sutures A and B are shown in Table 2 below.

**Table 2**

| Days elapsed (days) | Tensile strength (N) | |
|---|---|---|
| | Suture A | Suture B |
| 0 | 4.6 | 4.3 |
| 3 | 3.8 | 2.5 |
| 7 | 3.0 | 1.9 |

As a result of the above test, it was found that the suture loading the degrading enzyme was also varied in tensile strength with time by varying the amount of the enzyme to be mixed in the surface and inside thereof. It may be therefore considered that the rate of degradation of the suture can be controlled by regulating the amount of the degrading enzyme to be loaded.

### <Example 2>

### (Comparative example) (Suture C)

0.25 µL of heat-resistant chitinase (manufactured by Thermostable Enzyme Laboratory Co., Ltd.) having an active unit of 101 U/ml was added to and mixed with 4.5 ml of a mixture solution prepared by mixing a filament sizing oil agent (manufactured by Takemoto Oil & Fats Co., Ltd.) and ethanol with the weight ratio of the filament sizing oil agent to ethanol is 1:2 to prepare a coating solution. One suture (length: 200 mm) obtained in Comparative Example 1 was dipped in this coating solution for 5 minutes, and then, air-dried to remove ethanol.

### (Suture D)

A suture D was produced in the same manner as in the case of the suture C except that the amount of heat-resistant chitinase (manufactured by Thermostable Enzyme Laboratory Co., Ltd.) to be added was 25 µL.

The sutures C and D (each length: 200 mm) obtained above were dipped in 200 µ of a dipping solution having the same composition as that of Comparative Example 1 at 37°C for 5 minutes, washed, and dried to measure a variation in the tensile strength of the sutures with time. The tensile strength was measured in the same manner as in Comparative Example 1.

The results of measurement of the tensile strength of the sutures C and D are shown in Table 3 below.

**Table 3**

| Days elapsed (days) | Tensile strength | |
|---|---|---|
| | Suture C | Suture D |
| 0 | 5.9 | 5.9 |
| 3 | 4.8 | 4.1 |
| 7 | 4.1 | 3.1 |

It was found that the suture applied with a coating agent containing the degrading enzyme was also varied in tensile strength with time by varying the amount of the enzyme to be contained in the coating agent. It may be therefore considered that the rate of degradation of the suture can be controlled by regulating the amount of the degrading enzyme in the coating agent.

### (Comparative example) (Suture E)

0.25 µL of heat-resistant chitinase (manufactured by Thermostable Enzyme Laboratory Co., Ltd.) having an active unit of 101 U/ml was added to and mixed with 4.5 ml of a dipping solution having the same composition as that of Comparative Example 1. One suture (length: 200 mm) obtained in Comparative Example 1 was dipped in this coating solution for 5 minutes, and then, air-dried to remove water.

### (Suture F)

A suture F was produced in the same manner as in the case of the suture E except that the amount of heat-resistant chitinase (Thermostable Enzyme Laboratory Co., Ltd.) to be added was 25 µL.

The sutures E and F (each length: 200 mm) obtained in the above manner were dipped in 200 µ of a dipping solution having the same composition as that of Comparative Example 1 at 37°C for 5 minutes, washed, and dried to measure a variation in the tensile strength of the sutures with time. The tensile strength was measured in the same manner as in Comparative Example 1.

The results of measurement of the tensile strength of the sutures E and F are shown in Table 4 below.

**Table 4**

| Days elapsed (days) | Tensile strength | |
|---|---|---|
| | Suture E | Suture F |
| 0 | 5.9 | 5.9 |
| 3 | 3.8 | 2.7 |
| 7 | 3.1 | 1.7 |

It was found that the suture applied with the degrading enzyme was also varied in tensile strength with time by varying the amount of the enzyme to be attached. It may be therefore considered that the rate of degradation of the suture can be controlled by regulating the amount of the degrading enzyme to be attached.

## Claims

1. A bioabsorbable suture having a controlled rate of degradation, **characterized in that**
the suture is a multifilament yarn obtained by combining fibers containing one or two or more component (s) selected from the group consisting of chitin, chitosan, and derivatives thereof with a bioabsorbable synthetic polymer fiber and/or bioabsorbable fibers derived from a naturally-occurring polymer other than the fibers containing one or two or more component(s) selected from the group consisting of chitin, chitosan and derivatives thereof,
a degrading enzyme for the one or two or more component(s) selected from the group consisting of chitin, chitosan and derivatives thereof exists on a surface of the suture and in the suture, and
the suture is manufactured from monofilaments or multifilaments spun from a spinning raw solution containing the one or two or more component(s) selected from the group consisting of chitin, chitosan, and derivatives thereof and [a] the degrading enzyme.

2. A bioabsorbable suture having a controlled rate of degradation, **characterized in that**
the suture is a multifilament yarn obtained by combining fibers containing one or two or more component (s) selected from the group consisting of chitin, chitosan, and derivatives thereof with a bioabsorbable synthetic polymer fiber and/or bioabsorbable fibers derived from a naturally-occurring polymer other than the fibers containing one or two or more component(s) selected from the group consisting of chitin, chitosan and derivatives thereof, and
the suture is manufactured from fibers obtained by applying a coating agent containing a degrading enzyme for the one or two or more component (s) selected from the group consisting of chitin, chitosan, and derivatives thereof to at least a surface of
monofilaments or multifilaments spun from a spinning raw solution containing the one or two or more component(s) selected from the group consisting of chitin, chitosan, and derivatives thereof.

3. A bioabsorbable suture having a controlled rate of degradation, **characterized in that**
the suture is a multifilament yarn obtained by combining fibers containing one or two or more component (s) selected from the group consisting of chitin, chitosan, and derivatives thereof with a bioabsorbable synthetic polymer fiber and/or bioabsorbable fibers derived from a naturally-occurring polymer other than the fibers containing one or two or more component(s) selected from the group consisting of chitin, chitosan and derivatives thereof, and
the suture is manufactured from fibers obtained by attaching a degrading enzyme for the one or two or more component (s) selected from the group consisting of chitin, chitosan, and derivatives thereof to at least a surface of monofilaments or multifilaments spun from a spinning raw solution containing the one or two or more component(s) selected from the group consisting of chitin, chitosan, and derivatives thereof.

4. The bioabsorbable suture according to any one of claims 1 to 3,
**characterized in that** the degrading enzyme is a heat-resistant enzyme which is not deactivated under a temperature of at least 70°C.

5. The bioabsorbable suture according to claim 1,
**characterized in that** the suture is manufactured by mixing a spinning raw solution containing the one or two or more component(s) and the degrading enzyme, and a spinning raw solution containing the bioabsorbable synthetic polymer fiber and/or the bioabsorbable fibers, at a spinneret to spin both of the spinning raw solutions simultaneously.

6. The bioabsorbable suture according to claim 1,
**characterized in that** the suture is manufactured by twisting a filament obtained by spinning a spinning raw solution containing the one or two or more component(s) and the degrading enzyme, and a filament obtained by spinning a spinning raw solution containing the bioabsorbable synthetic polymer fiber and/or the bioabsorbable fibers.

7. The bioabsorbable suture according to any one of claims 2 to 3,
**characterized in that** the monofilaments or multifilaments are manufactured by mixing a spinning raw solution containing the one or two or more component(s), and a spinning raw solution containing the bioabsorbable synthetic polymer fiber and/or the bioabsorbable fibers, at a spinneret to spin both of the spinning raw solutions simultaneously.

8. The bioabsorbable suture according to any one of claims 2 to 3,
**characterized in that** the monofilaments or multifilaments are manufactured by twisting a filament obtained by spinning a spinning raw solution containing the one or two or more component(s), and a filament obtained by spinning a spinning raw solution containing the bioabsorbable synthetic polymer fiber and/or the bioabsorbable fibers.

## Patentansprüche

1. Biologisch absorbierbare Naht, die eine gesteuerte Abbaurate aufweist, **dadurch gekennzeichnet, dass**
die Naht ein Multifilamentgarn ist, das durch Kombinieren von Fasern, die eine oder zwei oder mehr Komponenten enthalten, die aus der Gruppe ausgewählt sind, die aus Chitin, Chitosan und Derivaten davon besteht, mit einer biologisch absorbierbaren Polymersynthesefaser und/oder mit biologisch absorbierbaren Fasern, die von einem anderen natürlich vorkommenden Polymer als die Fasern, die eine oder zwei oder mehr Komponenten enthalten, die aus der Gruppe ausgewählt sind, die aus Chitin, Chitosan und Derivaten davon besteht, abgeleitet sind, erhalten wird,
auf einer Oberfläche der Naht und in der Naht ein abbauendes Enzym für die eine oder zwei oder mehr Komponenten, die aus der Gruppe ausgewählt sind, die aus Chitin, Chitosan und Derivaten davon besteht, vorhanden ist, und
die Naht aus Monofilamenten oder Multifilamenten hergestellt wird, die aus einer Spinnrohlösung gesponnen werden, die die eine oder die zwei oder die mehr Komponenten, die aus der Gruppe ausgewählt sind, die aus Chitin, Chitosan und Derivaten davon besteht, und [ein] das abbauende Enzym enthält.

2. Biologisch absorbierbare Naht, die eine gesteuerte Abbaurate aufweist, **dadurch gekennzeichnet, dass**
die Naht ein Multifilamentgarn ist, das durch Kombinieren von Fasern, die eine oder zwei oder mehr Komponenten enthalten, die aus der Gruppe ausgewählt sind, die aus Chitin, Chitosan und Derivaten davon besteht, mit einer biologisch absorbierbaren Polymersynthesefaser und/oder mit biologisch absorbierbaren Fasern, die von einem anderen natürlich vorkommenden Polymer als die Fasern, die eine oder zwei oder mehr Komponenten enthalten, die aus der Gruppe ausgewählt sind, die aus Chitin, Chitosan und Derivaten davon besteht, abgeleitet sind, erhalten wird, und
die Naht aus Fasern hergestellt wird, die durch Auftragen eines Beschichtungsmittels, das ein abbauendes Enzym für die eine oder für die zwei oder für die mehr Komponenten, die aus der Gruppe ausgewählt sind, die aus Chitin, Chitosan und Derivaten davon besteht, enthält, auf wenigstens eine Oberfläche von Monofilamenten oder Multifilamenten, die aus einer Spinnrohlösung gesponnen werden, die die eine oder die zwei oder die mehreren Komponenten, die aus der Gruppe ausgewählt sind, die aus Chitin, Chitosan und Derivaten davon besteht, enthält, erhalten werden.

3. Biologisch absorbierbare Naht, die eine gesteuerte Abbaurate aufweist, **dadurch gekennzeichnet, dass**
die Naht ein Multifilamentgarn ist, das durch Kombinieren von Fasern, die eine oder zwei oder mehr Komponenten enthalten, die aus der Gruppe ausgewählt sind, die aus Chitin, Chitosan und Derivaten davon besteht, mit einer biologisch absorbierbaren Polymersynthesefaser und/oder mit biologisch absorbierbaren Fasern, die von einem anderen natürlich vorkommenden Polymer als die Fasern, die eine oder zwei oder mehr Komponenten enthalten, die aus der Gruppe ausgewählt sind, die aus Chitin, Chitosan und Derivaten davon besteht, abgeleitet sind, erhalten wird, und
die Naht aus Fasern hergestellt wird, die durch Anhaften eines abbauenden Enzyms für die eine oder für die zwei oder für die mehr Komponenten, die aus der Gruppe ausgewählt sind, die aus Chitin, Chitosan und Derivaten davon besteht, wenigstens an eine Oberfläche von Monofilamenten oder Multifilamenten, die aus einer Spinnrohlösung gesponnen werden, die die eine oder die zwei oder die mehr Komponenten enthält, die aus der Gruppe ausgewählt sind, die aus Chitin, Chitosan und Derivaten davon besteht, erhalten werden.

4. Biologisch absorbierbare Naht nach einem der Ansprüche 1 bis 3, **dadurch gekennzeichnet, dass** das abbauende Enzym ein wärmebeständiges Enzym ist, das unter einer Temperatur von wenigstens 70°C nicht deaktiviert wird.

5. Biologisch absorbierbare Naht nach Anspruch 1, **dadurch gekennzeichnet, dass** die Naht durch Mischen einer Spinnrohlösung, die die eine oder die zwei oder die mehr Komponenten und das abbauende Enzym enthält, und einer Spinnrohlösung, die die biologisch absorbierbare Polymersynthesefaser und/oder die biologisch absorbierbaren Fasern enthält, in einer Spinndüse zum gleichzeitigen Spinnen der beiden Spinnrohlösungen hergestellt wird.

6. Biologisch absorbierbare Naht nach Anspruch 1, **dadurch gekennzeichnet, dass** die Naht durch Verdrehen eines Filaments, das durch Spinnen einer Spinnrohlösung, die die eine oder die zwei oder die mehr Komponenten und das abbauende Enzym enthält, erhalten wird, und eines Filaments, das durch Spinnen einer Spinnrohlösung, die die biologisch absorbierbare synthetische Polymerfaser und/oder die biologisch absorbierbaren Fasern enthält, erhalten wird, hergestellt wird.

7. Biologisch absorbierbare Naht nach einem der Ansprüche 2 bis 3, **dadurch gekennzeichnet, dass** die Monofilamente oder Multifilamente durch Mischen einer Spinnrohlösung, die die eine oder die zwei oder die mehr Komponenten enthält, und einer Spinnrohlösung, die die biologisch absorbierbare synthetische Polymerfaser und/oder die biologisch absorbierbaren Fasern enthält, in einer Spinndüse zum gleichzeitigen Spinnen der beiden Spinnrohlösungen hergestellt werden.

8. Biologisch absorbierbare Naht nach einem der Ansprüche 2 bis 3, **dadurch gekennzeichnet, dass** die Monofilamente oder Multifilamente durch Verdrehen eines Filaments, das durch Spinnen einer Spinnrohlösung, die die eine oder die zwei oder die mehr Komponenten enthält, erhalten wird, und eines Filaments, das durch Spinnen einer Rohlösung, die die biologisch absorbierbare synthetische Polymerfaser und/oder die biologisch absorbierbaren Fasern enthält, erhalten wird, hergestellt werden.

## Revendications

1. Suture bioabsorbable ayant une vitesse contrôlée de dégradation, **caractérisée en ce que**
la suture est un fil multifilament obtenu en combinant des fibres contenant un composant, deux ou plus, choisi(s) dans le groupe constitué de la chitine, du chitosane, et des dérivés de ceux-ci avec une fibre de polymère synthétique bioabsorbable et/ou des fibres bioabsorbables dérivées d'un polymère d'origine naturelle autres que les fibres contenant un composant, deux ou plus, choisi(s) dans le groupe constitué de la chitine, du chitosane et des dérivés de ceux-ci,
une enzyme de dégradation pour le composant, les deux ou plus, choisi(s) dans le groupe constitué de la chitine, du chitosane et des dérivés de ceux-ci est présente sur une surface de la suture et dans la suture, et
la suture est fabriquée à partir de monofilaments ou de multifilaments filés à partir d'une solution brute de filage contenant le composant, les deux ou plus, choisi(s) dans le groupe constitué de la chitine, du chitosane, et des dérivés de ceux-ci et [a] l'enzyme de dégradation.

2. Suture bioabsorbable ayant une vitesse contrôlée de dégradation, **caractérisée en ce que**
la suture est un fil multifilament obtenu en combinant des fibres contenant un composant, deux ou plus, choisi(s) dans le groupe constitué de la chitine, du chitosane, et des dérivés de ceux-ci avec une fibre de polymère synthétique bioabsorbable et/ou des fibres bioabsorbables dérivées d'un polymère d'origine naturelle autres que les fibres contenant un composant, deux ou plus, choisi(s) dans le groupe constitué de la chitine, du chitosane et des dérivés de ceux-ci, et
la suture est fabriquée à partir de fibres obtenues en appliquant un agent de revêtement contenant une enzyme de dégradation pour composant, les deux ou plus, choisi(s) dans le groupe constitué de la chitine, du chitosane et des dérivés de ceux-ci à au moins une surface de monofilaments ou de multifilaments filés à partir d'une solution brute de filage contenant le composant, les deux ou plus, choisi(s) dans le groupe constitué de la chitine, du chitosane et des dérivés de ceux-ci.

3. Suture bioabsorbable ayant une vitesse contrôlée de dégradation, **caractérisée en ce que**
la suture est un fil multifilament obtenu en combinant des fibres contenant un composant, deux ou plus, choisi(s) dans le groupe constitué de la chitine, du chitosane, et des dérivés de ceux-ci avec une fibre de polymère synthétique bioabsorbable et/ou des fibres bioabsorbables dérivées d'un polymère d'origine naturelle autres que les fibres contenant un composant, les deux ou plus, choisi(s) dans le groupe constitué de la chitine, du chitosane et des dérivés de ceux-ci, et
la suture est fabriquée à partir de fibres obtenues en fixant une enzyme de dégradation pour le composant, les deux ou plus, choisi(s) dans le groupe constitué de la chitine, du chitosane, et des dérivés de ceux-ci à au moins une surface de monofilaments ou de multifilaments filés à partir d'une solution brute de filage contenant le composant, les deux ou plus, choisi(s) dans le groupe constitué de la chitine, du chitosane, et des dérivés de ceux-ci.

4. Suture bioabsorbable selon l'une quelconque des revendications 1 à 3,
**caractérisée en ce que** l'enzyme de dégradation est une enzyme thermorésistante qui n'est pas désactivée à une température d'au moins 70°C.

5. Suture bioabsorbable selon la revendication 1,
**caractérisée en ce que** la suture est fabriquée en mélangeant une solution brute de filage contenant le composant, les deux ou plus, et l'enzyme de dégradation, et une solution brute de filage contenant la fibre de polymère synthétique bioabsorbable et/ou les fibres bioabsorbables, au niveau d'une filière pour filer les deux solutions brutes de filage simultanément.

6. Suture bioabsorbable selon la revendication 1,
**caractérisée en ce que** la suture est fabriquée en tordant un filament obtenu par filage d'une solution brute de filage contenant le composant, les deux ou plus, et l'enzyme de dégradation, et un filament obtenu par filage d'une solution brute de filage contenant la fibre de polymère synthétique bioabsorbable et/ou les fibres bioabsorbables.

7. Suture bioabsorbable selon l'une quelconque des revendications 2 à 3,
**caractérisée en ce que** les monofilaments ou les multifilaments sont fabriqués en mélangeant une solution brute de filage contenant le composant, les deux ou plus, et une solution brute de filage contenant la fibre de polymère synthétique bioabsorbable et/ou les fibres bioabsorbables, au niveau d'une filière pour filer les deux solutions brutes de filage simultanément.

8. Suture bioabsorbable selon l'une quelconque des revendications 2 à 3,
**caractérisée en ce que** les monofilaments ou les multifilaments sont fabriqués en tordant un filament obtenu par filage d'une solution brute de filage contenant le composant, les deux ou plus, et un filament obtenu par filage d'une solution brute de filage contenant la fibre de polymère synthétique bioabsorbable et/ou les fibres bioabsorbables.
